# EUROPEAN PATENT SPECIFICATION

(11) **EP 1 419 146 B1**
(45) Date of publication and mention of the grant of the patent: **16.07.2008**
(21) Application number: 02796236.4
(22) Date of filing: 17.08.2002
(51) Int. Cl.: C07D 301/12, C07D 301/32

(54) **PROCESS FOR THE EPOXIDATION OF OLEFINS**
VERFAHREN ZUR EPOXIDIERUNG VON OLEFINEN
PROCEDE D'EPOXYDATION D'OLEFINES

(30) Priority: 22.08.2001 EP 01120165
(43) Date of publication of application: 19.05.2004
(73) Proprietor: Evonik Degussa GmbH, 40474 Düsseldorf (DE); Uhde GmbH, 44141 Dortmund (DE)
(72) Inventor: HAAS, Thomas, 60322 Frankfurt (DE); HOFEN, Willi, 63517 Rodenbach (DE); THIELE, Georg, 63450 Hanau (DE); WOELL, Wolfgang, 63477 Maintal (DE)
(74) Representative: Thiele, Georg Friedrich
(86) International application number: PCT/EP2002/009210
(87) International publication number: WO 2003/018567

(56) References cited:
- EP-A- 1 122 248
- DE-A- 19 835 907

## Description

The present invention relates to a process for the epoxidation of olefins, in particular to the working up of the product stream from the epoxidation reaction.

From EP-A 100 118 it is known that propene can be converted with hydrogen peroxide into propene oxide if titanium silicalite is used as catalyst. The reaction is preferably carried out in the presence of a water-miscible solvent in order to improve the solubility of propene in the reaction mixture. Preferably solvents are used that have a boiling point between the boiling points of propene oxide and water in order to be able to separate the solvent from the reaction mixture by a distillation stage and recycle it to the reaction. Methanol is preferably used as solvent.

From US-A 5,599,955 it is known that the reaction mixture which is obtained in the epoxidation reaction and which consists of propene oxide, propene, possibly propane, solvent and water can be separated by a succession of distillation steps, in which the mixture is separated in a first distillation step into an overhead product containing propene oxide, propene, and possibly propane, and into a bottom product containing the solvent and water. The efficient separation of propene oxide and solvent in a distillation step requires a large number of separation stages and a high reflux ratio in the distillation column.

WO-A 99/07690 describes a process for the purification of a methanol-containing product stream from the epoxidation of propene that also contains acetaldehyde as an impurity. In this case the crude product stream from the epoxidation is subjected to a fractional distillation, in which connection it is particularly important that methanol is present in sufficient amount in the overhead product in order to achieve a substantially complete transfer of acetaldehyde to the bottom product. To this end the concentration of methanol in the overhead product is 2-6 wt.%. A distillation column with 20-60 separation stages and a reflux ratio of between 10:1 and 30:1 is furthermore necessary in order to achieve the best possible quantitative separation of the acetaldehyde. This arrangement accordingly involves high investment and operating costs for the distillation column.

From US 5,849,938 it is known that in the distillative working up of the methanol-containing reaction mixture from the propene epoxidation, the difference in volatilities of propene oxide and methanol can be increased by carrying out the distillation as an extractive distillation using water or propylene glycol as extraction agent. The purpose of this extractive distillation is to separate methanol as well as further high boiling point impurities as quantitatively as possible from the desired product, namely propene oxide, in one distillation step. This requires a large number of separation stages and a high reflux ratio in the distillation column. In order to achieve the desired separation result, at least 10 theoretical trays, preferably 20-60 theoretical trays are required with a reflux ratio in the range from 5:1 to 15:1. The working examples disclose 25 or 50 theoretical trays and a reflux ratio of 9:1 for the extraction distillation column.

WO 00/07965 discloses a process for reacting an organic compound with a hydroperoxide especially for reacting propene with aqueous hydrogen peroxide in methanol as solvent, wherein the reaction product is subjected to a distillation step to separate a head product comprising propene, propene oxide and methanol from a bottom product comprising unreacted hydrogen peroxide. The distillation column having 15 theoretical trays is run at ambient pressure. The bottom stream is used as feed stream for a second reaction step.

In the known processes for the epoxidation of propene with H₂O₂ and titanium silicalite followed by distillative working up of the reaction mixture, due to the large number of separation steps and the high reflux ratio the residence time of propene oxide in the sections of the distillation column is long and there are also high concentrations of water and higher boiling point byproducts in the said sections, and accordingly the temperature is considerably higher than the boiling point of propene oxide under the distillation conditions. It has now been found that, as a result, there is an increased level of secondary reactions of propene oxide with water and other substances containing hydroxyl groups in the reaction mixture, which leads to undesirable losses of propene oxide. This disadvantage is particularly serious if the distillation is carried out under elevated pressure and thus at elevated temperature, which is advantageous for industrial exploitation since propene oxide can then be condensed with cooling water at the head of the distillation column and no expensive and energy-intensive cooling units have to be used.

This disadvantage of the known processes is exacerbated still further if the titanium silicalite catalyst used for the epoxidation reaction passes together with the reaction mixture into the separation column, since the catalyst also accelerates the undesirable secondary reactions of propene oxide with water and/or with other substances containing hydroxyl groups. If the epoxidation reaction is carried out with a suspended titanium silicalite catalyst, then in the known processes this catalyst must therefore be removed completely from the reaction mixture before the distillative separation of propene oxide and solvent. The separation of the catalyst at this point is particularly complicated since the separation is carried out in the presence of the highly volatile and carcinogenic substance propene oxide, and expensive and complicated industrial safety measures are therefore necessary. Also, precautions have to adopted in the known processes when using a fixed bed catalyst, for example by employing filtration, in order to prevent catalyst abrasion products settling in the distillation column and thereby causing product losses on account of the catalysis of the secondary reactions of propene oxide with water and/or other substances containing hydroxyl groups.

EP-A 1122248 discloses a process for the working up of a product stream from the epoxidation of olefins that contains olefin, olefin oxide, water-miscible organic solvent, hydrogen peroxide and water, by separating this product stream into an overhead product containing olefin, olefin oxide and organic solvent, and into a bottom product containing organic solvent, hydrogen peroxide and water, whereby the separation is carried out in a pre-evaporator with at most 5 theoretical separation stages at a pressure of 3 to 8 bar and 20 to 60% of the total amount of organic solvent introduced with the product stream is removed with the overhead product and the residue remains in the bottom product

Although the process described in EP-A 1122248 results in an considerable reduction of losses of epoxidation product compared to the hitherto known processes there is still a desire in industry to further improve the efficiency of the working up of the product stream from the epoxidation of olefins

### Subject of the invention:

This object is attained by a process for the working up of a product stream from the epoxidation of olefins that contains olefin, olefin oxide, water-miscible organic solvent, hydrogen peroxide and water by separating this product stream into an overhead product containing olefin, olefin oxide, and organic solvent, and into a bottom product containing organic solvent, hydrogen peroxide and water, wherein the separation takes place in a pre-evaporator with less than 10 theoretical separation stages and 20 to 60% of the total amount of organic solvent entrained in the product stream is removed with the overhead product, the balance remaining in the bottom product, whereby the separation in the pre-evaporator is conducted at a pressure from 1.5 to 2.9 bar, preferably 2 to 2.9 bar and the pre-evaporator is heated with the condensation heats of vapors resulting, from distillation steps of subsequent working-up stages.

This object is furthermore achieved by a process for the catalytic epoxidation of olefins in which the olefin is reacted in a reaction step with aqueous hydrogen peroxide in a water-miscible organic solvent in the presence of a titanium silicalite catalyst, the product stream from the reaction step being optionally added to a pressure release step and then worked up according to the aforedescribed process without prior distillative separation.

It has now been found that in the epoxidation of olefins with hydrogen peroxide and a titanium silicalite catalyst using an organic water-miscible solvent, the losses of olefin oxide in the distillative working up of the reaction mixture can be further reduced compared to the process disclosed in EP-A 1122248 if the processes according to the invention are employed. Furthermore it has been surprisingly discovered that by conducting the pre-evaporation within the pressure range of the process of the present invention decomposition of unreacted hydrogen peroxide is considerably reduced. Consequently the generation of molecular oxygen by peroxide decomposition is reduced. Therefore the likelihood of generating explosive compositions in any of the subsequent work-up stages is very low so that no extra precautions have to be taken to ensure safety of the process. Thus this measure improves the overall economics of the process.

A further advantage of the present process is, that the bottom stream comprising unreacted hydrogen peroxide from the pre-evaporation can be partially recycled to the reaction step.

Still a further advantage of the present invention compared to the teaching of EP-A 1122248 is the improved energy efficiency as will be discussed in some detail below.

### Description of the invention:

The processes according to the invention are particularly suitable for the epoxidation of olefins having two to six carbon atoms, propene being particularly preferred. The epoxidation reaction of olefins according to the invention is described hereinafter with the example of propene as preferred olefin.

The epoxidation reaction with hydrogen peroxide is carried out in the presence of a titanium silicalite catalyst in an organic water-miscible solvent. For the epoxidation of propene a solvent is preferably chosen whose boiling point is between the boiling points of propene oxide and water. Suitable solvents include, *inter alia,* alcohols, for example methanol, ethanol or tert.-butanol, ethers, for example tetrahydrofuran or 1,2-dimethoxyethane, and ketones, for example acetone. Methanol is preferably used as solvent.

Due to recycling of substances in the process, the solvent used may contain 0 to 20 wt.% of water. Hydrogen peroxide is used as an aqueous solution containing 10 to 90 wt.% of hydrogen peroxide. A hydrogen peroxide crude product obtained from the extraction step of the anthraquinone process and containing 30 to 45 wt.% of hydrogen peroxide is preferably used. Propene may be used mixed with propane in an amount of between 0 and 10 vol.% of propane.

In one embodiment of the invention the titanium silicalite catalyst is suspended in the reaction mixture during the reaction. The catalyst is then used in the form of a powder or in the form of a suspendable granular material that has been produced by forming in a manner known *per se*, for example by spray drying or fluidised bed granulation. When using a suspended catalyst, flow mixing reactors, for example stirred tank reactors or recycle reactors, as well as non-flow mixing reactors, for example tubular flow reactors, may be used for the reaction. A cascade consisting of one to three flow mixing reactors and a non-flow mixing reactor connected downstream is preferably used.

In another embodiment of the invention the titanium silicalite catalyst is used as a fixed bed over which a mixture of the feedstock materials is passed. The catalyst is then used in the form of formed bodies that have been produced in a manner known *per se,* for example by extrusion with the addition of binders. When using a fixed bed catalyst, reactors with bubble column characteristics are preferably used, i.e. a continuous liquid phase and a dispersed gaseous phase simultaneously flow through the reactor.

The epoxidation reaction is carried out at temperatures between 0 and 80 °C, preferably between 40 and 65°C, and at elevated pressures of 10 to 40 bar, preferably 15 to 30 bar under an atmosphere substantially consisting of propene. The propene is used in excess and the residence time in the reactor is chosen so that a hydrogen peroxide conversion of more than 90%, preferably more than 95%, is achieved. The amount of solvent used is preferably chosen so as to achieve a ratio of 1 to 5 parts by weight of solvent to one part by weight of aqueous hydrogen peroxide solution.

Before the working up stage the pressure of the reaction mixture is preferably released in a pressure release stage to the pressure employed in the working up of the propene oxide. Part of the propene dissolved in the reaction mixture and possibly propane is gassed out. The resultant gas is recompressed via a compressor to the pressure prevailing in the reactor and is returned to the reaction, the propene oxide still contained in the gas preferably being removed via an absorption column together with the solvent used for the reaction, before the compression.

The reaction mixture is then separated in a pre-evaporator into an overhead product containing propene, possibly propane, propene oxide and solvent, and into a bottom product containing solvent, water, higher boiling point byproducts, such as for example propylene glycol, unreacted hydrogen peroxide and possibly suspended titanium silicalite catalyst. The pre-evaporator according to the invention has less than 10, preferably at most 5 theoretical separation steps and is preferably designed so that the stripping section corresponds to a simple evaporation and the remaining separation effect is achieved in the rectification section. The pre-evaporator is operated at a reflux ratio of at most 1.5 and if desired may also be operated totally without reflux. The pressure in the pre-evaporator is chosen in the range from 1.5 to less than 3 bar in order to avoid decomposition of hydrogen peroxide. The pre-evaporator is operated according to the invention so that between 20 and 60% of the amount of solvent fed in with the reaction mixture is removed with the overhead product and the balance remains in the bottom product. In the operational procedure according to the invention more than 95%, typically more than 98% and preferably more than 99% of the propene oxide fed in is contained in the overhead product, and more than 90%, preferably more than 97% of the water fed in is contained in the bottom product.

The product stream fed to the pre-evaporator normally contains 0.5 - 20 wt.% of propene, 0 - 4 wt.% of propane, 5 - 35 wt.% of propene oxide, 35 - 80 wt.% of methanol, 5 - 40 wt.% of water, 0.1 - 8 wt.% of higher boiling point byproducts, 0.1 to 5 wt.% hydrogen peroxide and 0 - 5 wt.% of titanium silicalite catalyst. This product stream is separated in the process according to the invention into an overhead product containing 1 - 40 wt.% of propene, 0 - 10 wt.% of propane, 15 - 75 wt.% of propene oxide, 20 - 85 wt.% of methanol and 0 - 5 wt.% of water, and into a bottom product containing 0 - 2 wt.% of propene oxide, 30 - 80 wt.% of methanol, 15 - 65 wt.% of water, 0.1 - 10 wt.% of higher boiling point byproducts, 0.1 - 5 wt.% of hydrogen peroxide and 0 - 10 wt.% of titanium silicalite catalyst.

In one embodiment the overhead product is preferably only partially condensed and the uncondensed propene, possibly mixed with propane, is recompressed via a compressor to the pressure prevailing in the reaction part and is recycled to the reaction, the propene oxide still contained in the gas preferably being removed via an absorption column together with the solvent used for the reaction, before the compression, The propene still dissolved in the condensate and possibly propane are stripped out from the condensate in a C3 stripper and the stripped-out gas is recycled to the partial condenser. The mixture of propene oxide and solvent contained in the C3 stripper is separated by distillation into a propene oxide crude product, which can be purified further in a manner known *per se*, and the solvent, which is recycled to the epoxidation reaction.

According to an alternative embodiment the overhead product from the pre-evaporator is partially condensed in a first condenser and the gaseous effluent from the first condenser is condensed in a second condenser maintained at a temperature below the temperature of the first condenser. Preferably the temperature within the first condenser is maintained at 40-70°C and the temperature within the second condenser is maintained at 20-35°C. By using a two step condensation the amount of valuable propene oxide that can not be recovered is considerably reduced. Due to the low pressure in the pre-evaporation step in some instances cooling water without using a cooling unit may be insufficient in order to substantially condense the propene oxide from the overhead product. Therefore it may be advantageous to control the temperature within the first and the second condenser by using a cooling medium maintained at a temperature of 0 - 15°C.

A further advantage of the two step condensation is, that in case of using a cooling unit to maintain low temperatures of the cooling medium the energy consumption for cooling is reduced compared to a one step condensation.

When applying the two step condensation the condensates of both condenser are passed to a stripper to remove constituents having a boiling point that is lower than that of propene oxide, whereby the gaseous effluent from the stripper is partially condensed in the second condenser and the condensate is recycled to the stripper.

In a particularly preferred embodiment the mixture of propene oxide and solvent, preferably methanol, obtained from the C3 stripper is worked up further by extractive distillation to achieve as quantitative a separation as possible of the solvent. In this connection the mixture of propene oxide and methanol is added to the middle section of an extractive distillation column, preferably at a point corresponding to 1/3 of the total number of theoretical trays counting from the bottom, and a polar solvent with hydroxyl functionality and having a boiling point higher than that of methanol is added to the extractive distillation column at a point above the point at which the condensate enters, preferably at a point corresponding to 2/3 of the total number of theoretical trays counting from the bottom. The propene oxide crude product is distilled off at the head of the column and a mixture of methanol and the polar solvent is extracted as bottom product. The polar solvent is selected from water, glycols, glycol ethers and mixtures thereof. The preferred polar solvent is water since in this case the mixture of water and methanol can be recycled directly to the reaction step without further purification.

In order to achieve as complete a separation of the methanol as possible, a column with 25-100 theoretical separation steps and with a reflux ratio of 1-4 is already sufficient on account of the concentration of the propene oxide in the overhead product, the mathematical product of the number of separation steps and the reflux ratio typically being 75 to 125.

On account of the pre-evaporation according to the invention, according to the preferred embodiment of the process according to the invention only a very small reflux ratio for the extractive distillation step is still necessary in order to achieve the desired separation effect. Despite the two-stage procedure the operating costs for separating the water and solvent are thereby reduced compared to the prior art.

According to an especially preferred embodiment of the present invention the bottom stream comprising unreacted hydrogen peroxide from the pre-evaporation step is at least partially recycled to the epoxidation step thereby increasing the overall conversion of hydrogen peroxide. Preferably 20 to 80 % of the bottom stream is recycled. Preferably the bottom stream from the pre-evaporation step is subjected to further working-up stages for example to remove water or to remove high boiling side products prior to recycling the bottom stream in order to avoid accumulation of these products in the reaction mixture.

A particular advantage of the present process is the possibility of using an energy management that results in improved energy efficiency. Due to the low pressure of 2.9 bar at most in the pre-evaporator according to the process of the present invention and in the stripper according to a preferred embodiment of the present invention as described above the pre-evaporator and/or the stripper can be heated with the condensation heat of vapors resulting from subsequent distillation steps for example from the working-up stages of the bottom stream from the pre-evaporator or the crude propene oxide stream. Thereby preferably the vapors from a subsequent distillation step are condensed in a heat exchanger whereby the condensation heat is used to evaporate a part of the bottom stream of the pre-evaporator or the stripper. The vapor is re-introduced into the bottom part of the pre-evaporator or stripper to attain the desired bottom temperature.

In case methanol is used as solvent in the epoxidation process in the working-up of the bottom stream of the pre-evaporator a distillation is conducted under pressure and methanol is separated as head product. Thereby the pressure in this distillation step is selected so that the temperature of the head product is higher than the bottom temperature of the pre-evaporator and stripper respectively. Thus the condensation heat of the head product of said distillation step can be used to heat the pre-evaporator and/or stripper in the manner as described above.

By using the above described heat management the energy efficiency of the entire process can be further improved. Consequently selection of the pressure in the pre-evaporation step according to the present invention provides the possibility to increase the over all efficiency of the process.

A particularly preferred embodiment of the present invention relates to a process for the catalytic epoxidation of propene in which
a) in a reaction step the propene is reacted with aqueous hydrogen peroxide in methanol in the presence of a titanium silicalite catalyst,
b) the product stream from the reaction step is optionally passed to a pressure release step, and
c) the product stream is then separated, without prior distillative separation, in a pre-evaporator having less than 10 theoretical separation steps at a pressure of 1.5 to 2.9 bar into an overhead product containing propene, propene oxide and methanol, and into a bottom product containing methanol and water, 20 to 60% of the total amount of methanol introduced into the product stream being removed with the overhead product and the residue remaining in the bottom product, whereby the pre-evaporator is heated with the condensation heat of vapors resulting from distillation steps of subsequent working-up stages,
d) the overhead product from step c) is at least partially condensed, the condensate containing, optionally after stripping out propene and any propane present

| | |
|---|---|
| 0 12 | wt.% propene, |
| 0 - 5 | wt.% propane, |
| 15 - 75 | wt.% propene oxide, |
| 25 - 85 | wt.% methanol and |
| 0 - 8 | wt.% water, and |

e) the condensate from step d) is subjected to an extractive distillation, wherein
e1) the condensate is added to a middle section of an extractive distillation column,
e2) a polar solvent with hydroxyl functionality and having a boiling point higher than that of methanol is added to the extractive distillation column at a point above the point at which the condensate enters,
e3) propene oxide is distilled off at the head of the column, and
e4) a bottom product containing methanol and the polar solvent is removed.

f) a part of the bottom product from step c) optionally after partially removing water is recycled to the reaction step a).

When using a suspended titanium silicalite catalyst the catalyst is recovered from the bottom product of the pre-evaporator by solid/liquid separation, for example by filtration or centrifugation, in which connection the solid/liquid separation can be carried out as desired either before or after the recovery of the solvent. A separation of the catalyst at this point of the process is particularly advantageous since the propene oxide, which represents a health hazard, has at this point already been separated and less stringent requirements are therefore placed on industrial safety, which considerably simplifies the overall process and makes it much more cost-effective.

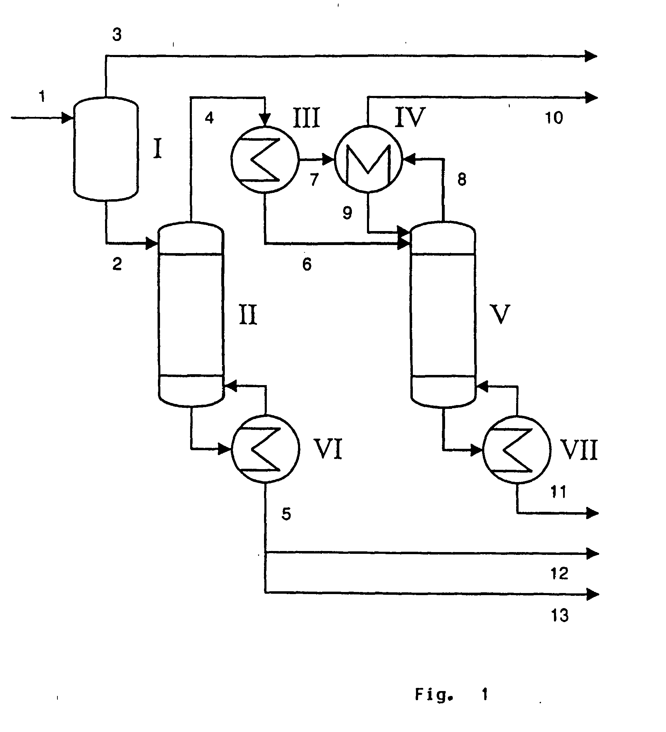

The present invention will be explained in more detail with reference to Fig. 1 and a working example.

### Example:

Fig. 1 illustrates the working up of the reaction mixture for a particularly preferred embodiment of the invention using a fixed bed catalyst and methanol as solvent. The reaction mixture 1 obtained from the epoxidation reaction step comprises 20.4 wt.% propene, 2 wt.% propane, 13.4 wt.% propene oxide, 0.1 wt.% low-boilers, 47.7 wt.% methanol, 15.5 wt.% water, 0.5 wt.% hydrogen peroxide and 1.1 wt.% high-boilers. The flow rate is 9.3 kg/h. The reaction mixture is directed to a pressure release unit (I) wherein the pressure is released from the reaction pressure (15-30 bar) to 2 bar. The gas stream 3 leaving the pressure release unit (I) comprising 81 wt.% propene, 7 wt.% propane, 8 wt.% propene oxide and 4 wt.% methanol (flow rate = 0.83 kg/h) is recycled to the reaction stage. The liquid effluent 2 from the pressure release unit (I) comprising 14.3 wt.-% propene, 1.5 wt.-% propane, 13.9 wt.-% propene oxide, 51.8 wt.-% methanol, 17 wt.-% water, 0.5 wt.-% hydrogen peroxide and 1.3 wt.-% high-boilers (flow-rate = 8.5 kg/h)is separated in a pre-evaporation column (II) according to the invention into an overhead stream 4 containing 34.5 wt.% propene, 3.4 wt.-% propane, 34.3 wt.% propene oxide, 25.6 wt.% methanol and 2.3 wt.-% water (flow rate 3.46 kg/h), and a bottom stream 5 containing 69.8 wt.-% methanol, 27.1 wt.-% water, 0.8 wt.-% hydrogen peroxide and 2.2 wt.-% propylene glycol monomethyl ether and other high boiling compounds (flow rate = 5.08 kg/h). Overhead stream 4 is separated in a first condenser (III) maintained at 60°C into a condensate 6 comprising 4.1 wt.-% propene, 0.4 wt.-% propane, 44.7 wt.-% propene oxide, 46.8 wt.-% methanol and 4.4 wt.-% water (flow rate 1.76 kg/h) and a gaseous effluent 7 comprising 66 wt.-% propene, 6.5 wt.-% propane, 23.6 wt.-% propene oxide, 3.8 wt.-% methanol and 0.1 wt.-% water (flow rate 1.7 kg/h). The condensate 6 is fed to the head of a C3-stripper (V). The gaseous effluent 8 of the C3-stripper comprising 31.2 wt.-% propene, 3.5 wt.-% propane, 60.8 wt.-% propene oxide and 4.4 wt.-% methanol (flow rate = 0.52 kg/h) and the gaseous effluent 7 from the first condenser (III) are combined and separated in a second condenser (IV) maintained at 30°C in a gaseous effluent 10 comprising 79.5 wt.-% propene, 7.8 wt.-% propane, 11.9 wt.-% propene oxide and 0.6 wt.-% methanol (flow rate 1.5 kg/h) that is recycled to the reaction stage and a condensate 9 comprising 13.3 wt.-% propene, 1.5 wt.-% propane, 74.1 wt.-% propene oxide, 10.8 wt.-% methanol and 0.4 wt.-% water that is recycled to the C3-stripper (V). The bottom stream 11 comprising 50.9 wt.-% propene oxide, 44.6 wt.-% methanol and 4.5 wt.-% water from the C3-stripper (V) (flow rate = 1.97 kg/h) is the crude propene oxide that is subjected to further purification steps. The bottom stream 5 from the pre-evaporation column (II) can be divided in two partial streams 12 and 13, whereby stream 12 is directly recycled to the reaction stage whereas stream 13 is subjected to subsequent purification steps. Further the bottom streams 5, 11 from the pre-evaporator II and the stripper V are partially evaporated in a heat exchanger VI, VII, whereby the heat for evaporation is provided by the condensation of vapors from subsequent distillation steps in the purification of stream 13 (not shown). The evaporated part of the bottom stream 5,11 are reintroduced into the pre-evaporator II and the stripper V respectively in order to attain the desired bottom temperature.

The process according to the invention has the advantage compared to the prior art that in the working up the duration of the thermal stresses to which the olefin oxide is subjected in the presence of water and other potential reactants is substantially shorter and therefore the loss of olefin oxide by secondary reactions and the decomposition of unreacted hydrogen peroxide in the working up is significantly reduced.

The invention also has the advantage that the separation of the propene oxide from methanol and water can be achieved with smaller reflux ratios in the columns than in the prior art, which leads to savings in operating costs. With the extractive distillation that is preferably used to separate propene oxide and methanol in the crude propene oxide stream using water as extraction agent there is also the advantage, compared to the prior art, that the methanol-water mixture obtained in the bottom of the column can be returned as solvent directly to the epoxidation process, with the result that no separate distillation column is required to recover the extraction agent.

When using a suspended titanium silicalite catalyst it is possible with the process according to the invention, in contrast to the prior art, to separate the propene oxide from the reaction mixture before the recovery of the catalyst by solid/liquid separation takes place. Considerable savings in the necessary industrial safety measures are possible thanks to the solid/liquid separation in the absence of the carcinogenic propene oxide.

Finally the process of the present invention provides the possibility to use an integrated heat management in order to improve energy efficiency.

## Claims

1. Process for the working up of a product stream from the epoxidation of olefins that contains olefin, olefin oxide, water-miscible organic solvent, hydrogen peroxide and water, by separating this product stream into an overhead product containing olefin, olefin oxide and organic solvent, and into a bottom product containing organic solvent, hydrogen peroxide and water, in a pre-evaporator with less than 10 theoretical separation stages, whereby 20 to 60% of the total amount of organic solvent introduced with the product stream is removed with the overhead product and the balance remains in the bottom product, **characterized in that** the separation is carried out at a pressure of 1.5 to 2.9 bar and the pre-evaporator is heated with the condensation heat of vapors resulting from distillation steps of subsequent working-up stages.

2. Process according to claim 1, **characterized in that** the reflux ratio in the pre-evaporator is at most 1.5.

3. Process according to one of the preceding claims, **characterized in that** more than 95%, preferably more than 98% and particularly preferably more than 99% of the entrained olefin oxide is removed with the overhead product, and more than 90%, preferably more than 97% of the entrained water is removed with the bottom product.

4. Process according to one of the preceding claims, **characterized in that** the product stream from the epoxidation of olefins comprises 0.1 to 5 wt-% preferably 0.3 to 3 wt-% hydrogen peroxide.

5. Process according to one of the preceding claims, **characterized in that** the pressure is from 2 to 2.9 bar.

6. Process for the catalytic epoxidation of olefins in which in a reaction step the olefin is reacted with aqueous hydrogen peroxide in an organic water-miscible solvent in the presence of a titanium silicalite catalyst, wherein the product stream from the reaction step is optionally fed to a pressure release step and is then worked up, without prior distillative separation, according to the process of one of claims 1 to 5.

7. Process according to claim 6 **characterized in that** the olefin is selected from a C₂-C₆ olefin and is preferably propene, and the solvent is selected from alcohols, ethers and ketones, and is preferably methanol.

8. Process according to claims 6 **characterized in that** the product stream fed to the pre-evaporator contains:
| | |
|---|---|
| 0.5 - 20 | wt.% propene |
| 0 - 4 | wt.% propane |
| 5 - 35 | wt.% propene oxide |
| 35 - 80 | wt.% methanol |
| 5 - 40 | wt.% water |
| 0.1 - 5 | wt.% hydrogen peroxide |
| 0.1 - 8 | wt.% byproducts |
| 0 - 5 | wt.% titanium silicalite catalyst, |
the overhead product from the pre-evaporator contains
| | |
|---|---|
| 1 - 40 | wt.% propene |
| 0 - 10 | wt.% propane |
| 15 - 75 | wt.% propene oxide |
| 20 - 85 | wt.% methanol |
| 0 - 5 | wt.% water |
and the bottom product from the pre-evaporator contains
| | |
|---|---|
| 0 - 2 | wt.% propene oxide |
| 30 - 80 | wt.% methanol |
| 15 - 65 | wt.% water |
| 0.1 - 5 | wt.% hydrogen peroxide |
| 0.1 - 10 | wt.% byproducts |
| 0 - 10 | wt.% titanium silicalite catalyst. |

9. Process according to one of claims 6 to 8, **characterized in that** the overhead product from the pre-evaporator is at least partially condensed, constituents having a boiling point that is lower than that of olefin oxide are optionally stripped from the condensate in a stripper, and the condensate is then subjected to an extractive distillation.

10. Process according to one of claims 6 to 8, **characterized in that** the overhead product from the pre-evaporator is partially condensed in a first condenser and the gaseous effluent from the first condenser is condensed in a second condenser maintained at a temperature below the temperature of the first condenser.

11. Process of claim 10, **characterized in that** the temperature the first condenser is maintained at 40-70°C and the temperature of the second condenser is maintained at 20-35°C.

12. Process of any of claims 10 and 11, **characterized in that** the condensates of both condenser are passed to a stripper to remove constituents having a boiling point that is lower than that of olefin oxide, whereby the gaseous effluent from the stripper is partially condensed in the second condenser and the condensate is recycled to the stripper.

13. Process of any of claims 9-12 **characterized in that** the stripper is heated with the condensation heat of vapors resulting from distillation steps of subsequent working-up stages.

14. The process of claim 13, **characterized in that** the stripped condensate is subjected to an extractive distillation.

15. Process according to one of claims 6 to 14, **characterized in that** the bottom stream from the pre-evaporator comprising hydrogen peroxide is at least partially recycled to the reaction step.

16. Process of claim 15, **characterized in that** water is partially removed from the bottom stream prior to recycling to the reaction step.

17. Process of one of the claims 6-16, **characterized in that** the titanium silicalite catalyst is present suspended in the reaction mixture.

18. Process according to claim 17, **characterized in that** the bottom product from the pre-evaporator contains titanium silicalite catalyst that is separated by solid/liquid separation.

19. Process according to one of claims 6 to 16, **characterized in that** the titanium silicalite catalyst is present as a fixed bed.

20. Process for the catalytic epoxidation of propene, in which
a) in a reaction step the propene is reacted with aqueous hydrogen peroxide in methanol in the presence of a titanium silicalite catalyst,
b) the product stream from the reaction step is optionally passed to a pressure release step, and
c) the product stream is then separated, without prior distillative separation, in a pre-evaporator having less than 10 theoretical separation stages at a pressure of 1.5 to 2,9 bar into an overhead product containing propene, propene oxide and methanol, and into a bottom product containing methanol and water, 20 to 60% of the total amount of methanol introduced with the product stream being removed with the overhead product and the residue remaining in the bottom product, whereby the pre-evaporator is heated with the condensation heat of vapors resulting from distillation steps of subsequent working-up stages,
d) the overhead product from step c) is at least partially condensed, the condensate containing, optionally after stripping out propene and any propane present
| | |
|---|---|
| 0 - 12 | wt.% propene, |
| 0 - 5 | wt.% propane, |
| 15 - 75 | wt.% propene oxide, |
| 25 - 85 | wt.% methanol and |
| 0 - 8 | wt.% water, and |
e) the condensate from step d) is subjected to an extractive distillation, wherein
e1) the condensate is added to a middle section of an extractive distillation column,
e2) a polar solvent with hydroxyl functionality and having a boiling point that is higher than that of methanol is added to the extractive distillation column at a point above the point at which the condensate enters,
e3) propene oxide is distilled off at the head of the column,
e4) a bottom product containing methanol and the polar solvent is removed, and
f) a part of the bottom product from step c) optionally after partially removing water is recycled to the reaction step a).

21. Process according to claim 20, **characterized in that** the polar solvent is selected from water, glycols, glycol ethers and mixtures thereof.

## Patentansprüche

1. Verfahren zur Aufarbeitung eines Produktstroms aus der Epoxidierung von Olefinen, der Olefin, Olefinoxid, wassermischbares organisches Lösungsmittel, Wasserstoffperoxid und Wasser enthält, durch Auftrennen dieses Produktstroms in ein Kopfprodukt, das Olefin, Olefinoxid und organisches Lösungsmittel enthält, und in ein Sumpfprodukt, das organisches Lösungsmittel, Wasserstoffperoxid und Wasser enthält, in einem Vorverdampfer mit weniger als 10 theoretischen Trennstufen, wobei 20 bis 60 % der Gesamtmenge des mit dem Produktstrom zugeführten organischen Lösungsmittels mit dem Kopfprodukt entfernt werden und der Rest im Sumpfprodukt verbleibt, **dadurch gekennzeichnet, dass** die Trennung bei einem Druck von 1,5 bis 2,9 bar durchgeführt wird und der Vorverdampfer mit der Kondensationswärme von Dämpfen, die aus den Destillationsschritten von nachfolgenden Aufarbeitungsstufen resultieren, beheizt wird.

2. Verfahren gemäß Anspruch 1, **dadurch gekennzeichnet, dass** das Rücklaufverhältnis in dem Vorverdampfer höchstens 1,5 beträgt.

3. Verfahren gemäß einem der vorhergehenden Ansprüche, **dadurch gekennzeichnet, dass** mehr als 95 %, vorzugsweise mehr als 98 %, und besonders bevorzugt mehr als 99 % des mitgeführten Olefinoxids mit dem Kopfprodukt entfernt werden und mehr als 90 %, vorzugsweise mehr als 97 % des mitgeführten Wassers mit dem Sumpfprodukt entfernt werden.

4. Verfahren gemäß einem der vorhergehenden Ansprüche, **dadurch gekennzeichnet, dass** der Produktstrom aus der Epoxidierung von Olefinen 0,1 bis 5 Gew.-%, vorzugsweise 0,3 bis 3 Gew.-% Wasserstoffperoxid umfasst.

5. Verfahren gemäß einem der vorhergehenden Ansprüche, **dadurch gekennzeichnet, dass** der Druck 2 bis 2,9 bar beträgt.

6. Verfahren zur katalytischen Epoxidierung von Olefinen, in welchem in einem Reaktionsschritt das Olefin mit wässrigem Wasserstoffperoxid in einem organischen wassermischbaren Lösungsmittel in Gegenwart eines Titansilicalitkatalysators umgesetzt wird, wobei der Produktstrom aus dem Reaktionsschritt gegebenenfalls einem Druckentspannungsschritt zugeführt wird und dann, ohne vorherige destillative Trennung, gemäß dem Verfahren von einem der Ansprüche 1 bis 5 aufgearbeitet wird.

7. Verfahren gemäß Anspruch 6, **dadurch gekennzeichnet, dass** das Olefin aus einem C₂-C₆-Olefin gewählt wird und vorzugsweise Propen ist und das Lösungsmittel aus Alkoholen, Ethern und Ketonen gewählt wird und vorzugsweise Methanol ist.

8. Verfahren gemäß Anspruch 6, **dadurch gekennzeichnet, dass** der in den Vorverdampfer eingespeiste Produktstrom
| | |
|---|---|
| 0,5 - 20 | Gew.-% Propen |
| 0 - 4 | Gew.-% Propan |
| 5 - 35 | Gew.-% Propenoxid |
| 35 - 80 | Gew.-% Methanol |
| 5 - 40 | Gew.-% Wasser |
| 0,1 - 5 | Gew.-% Wasserstoffperoxid |
| 0,1 - 8 | Gew.-% Nebenprodukte |
| 0 - 5 | Gew.-% Titansilicalitkatalysator |
enthält, das Kopfprodukt aus dem Vorverdampfer
| | |
|---|---|
| 1 - 40 | Gew.-% Propen |
| 0 - 10 | Gew.-% Propan |
| 15 - 75 | Gew.-% Propenoxid |
| 20 - 85 | Gew.-% Methanol |
| 0 - 5 | Gew.-% Wasser |
enthält und das Sumpfprodukt aus dem Vorverdampfer
| | |
|---|---|
| 0 - 2 | Gew.-% Propenoxid |
| 30 - 80 | Gew.-% Methanol |
| 15 - 65 | Gew.-% Wasser |
| 0,1 - 5 | Gew.-% Wasserstoffperoxid |
| 0,1 - 10 | Gew.-% Nebenprodukte |
| 0 - 10 | Gew.-% Titansilicalitkatalysator |
enthält.

9. Verfahren gemäß einem der Ansprüche 6 bis 8, **dadurch gekennzeichnet, dass** das Kopfprodukt aus dem Vorverdampfer zumindest teilweise kondensiert wird, Bestandteile mit einem Siedepunkt, der niedriger ist als derjenige von Olefinoxid, gegebenenfalls aus dem Kondensat in einem Austreiber ausgetrieben werden und das Kondensat dann einer Extraktivdestillation unterzogen wird.

10. Verfahren gemäß einem der Ansprüche 6 bis 8, **dadurch gekennzeichnet, dass** das Kopfprodukt aus dem Vorverdampfer teilweise in einem ersten Kondensator kondensiert wird und das Abgas aus dem ersten Kondensator in einem zweiten Kondensator, welcher auf einer Temperatur unterhalb der Temperatur des ersten Kondensators gehalten wird, kondensiert wird.

11. Verfahren gemäß Anspruch 10, **dadurch gekennzeichnet, dass** die Temperatur des ersten Kondensators auf 40 - 70°C gehalten wird und die Temperatur des zweiten Kondensators auf 20 - 35°C gehalten wird.

12. Verfahren gemäß einem der Ansprüche 10 und 11, **dadurch gekennzeichnet, dass** die Kondensate beider Kondensatoren zur Entfernung von Bestandteilen mit einem Siedepunkt, der niedriger ist als derjenige von Olefinoxid, einem Austreiber zugeführt werden, wobei das Abgas aus dem Austreiber teilweise in dem zweiten Kondensator kondensiert wird und das Kondensat in den Austreiber zurückgeführt wird.

13. Verfahren gemäß einem der Ansprüche 9 - 12, **dadurch gekennzeichnet, dass** der Austreiber mit der Kondensationswärme von Dämpfen, die aus den Destillationsschritten nachfolgender Aufarbeitungsschritte resultieren, beheizt wird.

14. Verfahren gemäß Anspruch 13, **dadurch gekennzeichnet, dass** das ausgetriebene Kondensat einer Extraktivdestillation unterworfen wird.

15. Verfahren gemäß einem der Ansprüche 6 bis 14, **dadurch gekennzeichnet, dass** der Sumpfstrom aus dem Vorverdampfer, welcher Wasserstoffperoxid umfasst, zumindest teilweise in den Reaktionsschritt zurückgeführt wird.

16. Verfahren gemäß Anspruch 15, **dadurch gekennzeichnet, dass** Wasser vor der Rückführung in dem Reaktionsschritt teilweise aus dem Sumpfstrom entfernt wird.

17. Verfahren gemäß einem der Ansprüche 6 - 16, **dadurch gekennzeichnet, dass** der Titansilicalitkatalysator suspendiert in der Reaktionsmischung vorliegt.

18. Verfahren gemäß Anspruch 17, **dadurch gekennzeichnet, dass** das Sumpfprodukt aus dem Vorverdampfer Titansilicalitkatalysator enthält, der durch Fest/Flüssig-Trennung abgetrennt wird.

19. Verfahren gemäß einem der Ansprüche 6 bis 16, **dadurch gekennzeichnet, dass** der Titansilicalitkatalysator als ein Festbett vorliegt.

20. Verfahren zur katalytischen Epoxidierung von Propen, in dem:
a) in einer Reaktionsschritt das Propen mit wässrigem Wasserstoffperoxid in Methanol in Gegenwart eines Titansilicalitkatalysators umgesetzt wird;
b) der Produktstrom aus dem Reaktionsschritt wahlweise einem Druckentspannungsschritt zugeführt wird; und
c) der Produktstrom danach ohne vorherige destillative Trennung in einem Vorverdampfer mit weniger als 10 theoretischen Trennstufen bei einem Druck von 1,5 bis 2,9 bar in ein Kopfprodukt, das Propen, Propenoxid und Methanol enthält, und ein Sumpfprodukt, das Methanol und Wasser enthält, aufgetrennt wird, wobei 20 bis 60 % der Gesamtmenge des mit dem Produktstrom eingeführten Methanols mit dem Kopfprodukt entfernt werden und der Rest im Sumpfprodukt verbleibt, wobei der Vorverdampfer mit der Kondensationswärme von Dämpfen, die aus Destillationsschritten von nachfolgenden Aufarbeitungsstufen resultieren, erwärmt wird;
d) das Kopfprodukt von Schritt c) zumindest teilweise kondensiert wird und das Kondensat, wahlweise nach Austreiben von Propen und vorhandenem Propan,
| | |
|---|---|
| 0 - 12 | Gew.-% Propen |
| 0 - 5 | Gew.-% Propan |
| 15 - 75 | Gew.-% Propenoxid |
| 25 - 85 | Gew.-% Methanol und |
| 0 - 8 | Gew.-% Wasser |
enthält; und
e) das Kondensat von Schritt d) einer Extraktivdestillation unterworfen wird, wobei:
e1) das Kondensat einem mittleren Abschnitt einer Extraktivdestillationskolonne zugeführt wird,
e2) ein polares Lösungsmittel mit Hydroxylfunktionalität und mit einem Siedepunkt, der höher ist als derjenige von Methanol, der Extraktivdestillationskolonne an einem Punkt oberhalb des Punktes, an welchem das Kondensat eintritt, zugeführt wird,
e3) Propenoxid am Kopf der Kolonne abdestilliert wird,
e4) ein Methanol und das polare Lösungsmittel enthaltendes Sumpfprodukt entfernt wird; und
f) ein Teil des Sumpfprodukts von Schritt c), wahlweise nach teilweiser Entfernung von Wasser, in den Reaktionsschritt a) zurückgeführt wird.

21. Verfahren gemäß Anspruch 20, **dadurch gekennzeichnet, dass** das polare Lösungsmittel aus Wasser, Glykolen, Glykolethern und Mischungen davon gewählt wird.

## Revendications

1. Procédé de traitement d'un flux de produit issu de l'époxydation d'oléfines qui contient une oléfine, un oxyde d'oléfines, un solvant organique miscible à l'eau, du peroxyde d'hydrogène et de l'eau, par séparation de ce flux de produit en un produit de tête contenant une oléfine, un oxyde d'oléfines et un solvant organique, et en un produit de fond contenant un solvant organique, du peroxyde d'hydrogène et de l'eau, dans un pré-évaporateur ayant moins de 10 étapes de séparation théoriques, 20 à 60% de la quantité totale de solvant organique introduite avec le flux de produit étant éliminée avec le produit de tête et le restant subsiste dans le produit de fond, **caractérisé en ce que** la séparation est effectuée à une pression de 1,5 à 2,9 bars et **en ce que** le pré-évaporateur est chauffé à l'aide de la chaleur de condensation des vapeurs résultant des étapes de distillation de phases subséquentes de traitement.

2. Procédé selon la revendication 1, **caractérisé en ce que** le rapport de reflux dans le pré-évaporateur est au plus de 1,5.

3. Procédé selon l'une quelconque des revendications précédentes, **caractérisé en ce que** plus de 95%, de préférence, plus de 98% et particulièrement, de préférence, plus de 99% de l'oxyde d'oléfines entraîné est éliminé avec le produit de tête et **en ce que** plus de 90%, de préférence, plus de 97% de l'eau entraînée est éliminée avec le produit de fond.

4. Procédé selon l'une quelconque des revendications précédentes, **caractérisé en ce que** le flux de produit provenant de l'époxydation d'oléfines comprend de 0,1 à 5% en poids, de préférence, de 0,3 à 3% en poids de peroxyde d'hydrogène.

5. Procédé selon l'une quelconque des revendications précédentes, **caractérisé en ce que** la pression va de 2 à 2,9 bars.

6. Procédé d'époxydation catalytique d'oléfines dans lequel dans une étape de réaction l'oléfine est mise en réaction avec du peroxyde d'hydrogène aqueux dans un solvant organique miscible à l'eau en présence d'un catalyseur de silicalite de titane, le flux de produit provenant de l'étape de réaction étant alimenté en option à une étape de décompression et est alors soumis à traitement, sans séparation par distillation antérieure, conformément au procédé selon l'une quelconque des revendications 1 à 5.

7. Procédé selon la revendication 6, **caractérisé en ce que** l'oléfine est sélectionnée d'une oléfine en C₂-C₆ et est, de préférence, le propène et **en ce que** le solvant est sélectionné parmi les alcools, les éthers et les cétones et est, de préférence, le méthanol.

8. Procédé selon la revendication 6, **caractérisé en ce que** le flux de produit alimenté au pré-évaporateur contient:
0,5 - 20 % en poids de propène
0 - 4 % en poids de propane
5 - 35 % en poids d'oxyde de propène
35 - 80 % en poids de méthanol
5 - 40 % en poids d'eau
0,1 - 5 % en poids de peroxyde d'hydrogène
0,1 - 8 % en poids de produits secondaires
0 - 5 % en poids de catalyseur de silicalite de titane,
**en ce que** le produit de tête en provenance du pré-évaporateur contient:
1 - 40 % en poids de propène
0 - 10 % en poids de propane
15 - 75 % en poids d'oxyde de propène
20 - 85 % en poids de méthanol
0 - 5 % en poids d'eau,
**en ce que** le produit de fond en provenance du pré-évaporateur contient:
0 - 2 % en poids d'oxyde de propène
30 - 80 % en poids de méthanol
15 - 65 % en poids d'eau
0,1 - 5 % en poids de peroxyde d'hydrogène
0,1 - 10 % en poids de produits secondaires
0 - 10 % en poids de catalyseur de silicalite de titane.

9. Procédé selon l'une quelconque des revendications 6 à 8, **caractérisé en ce que** le produit de tête en provenance du pré-évaporateur est au moins partiellement condensé, **en ce que** les constituants ayant un point d'ébullition qui est inférieur à celui de l'oxyde d'oléfines sont en option éliminés par stripage du condensat dans un dispositif de stripage et **en ce que** le condensat est alors soumis à une distillation extractive.

10. Procédé selon l'une quelconque des revendications 6 à 8, **caractérisé en ce que** le produit de tête en provenance du pré-évaporateur est partiellement condensé dans un premier condenseur et **en ce que** l'effluent gazeux en provenance du premier condenseur est condensé dans un second condenseur maintenu à une température en-dessous de la température du premier condenseur.

11. Procédé selon la revendication 10, **caractérisé en ce que** la température du premier condenseur est maintenue à la valeur de 40-70°C et **en ce que** la température du second condenseur est maintenue à la valeur de 20-35°C.

12. Procédé selon l'une quelconque des revendications 10 et 11, **caractérisé en ce que** les condensats des deux condenseurs sont acheminés vers un dispositif de stripage pour éliminer les constituants ayant un point d'ébullition qui est inférieur à celui de l'oxyde d'oléfines, de sorte que l'effluent gazeux en provenance du dispositif de stripage est partiellement condensé dans le second condenseur et le condensat est recyclé au dispositif de stripage.

13. Procédé selon l'une quelconque des revendications 9-12, **caractérisé en ce que** le dispositif de stripage est chauffé à l'aide de la chaleur de condensation des vapeurs résultant des étapes de distillation des phases subséquentes de traitement.

14. Procédé selon la revendication 13, **caractérisé en ce que** le condensat ayant subi un stripage est soumis à une distillation extractive.

15. Procédé selon l'une quelconque des revendications 6 à 14, **caractérisé en ce que** le flux de fond en provenance du pré-évaporateur comprenant le peroxyde d'hydrogène est au moins partiellement recyclé à l'étape de réaction.

16. Procédé selon la revendication 15, **caractérisé en ce que** l'eau est partiellement éliminée du flux de fond avant le recyclage à l'étape de réaction.

17. Procédé selon l'une quelconque des revendications 6-16, **caractérisé en ce que** le catalyseur de silicalite de titane est présent en suspension dans le mélange réactionnel.

18. Procédé selon la revendication 17, **caractérisé en ce que** le produit de fond en provenance du pré-évaporateur contient un catalyseur de silicalite de titane qui est séparé par séparation solide/liquide.

19. Procédé selon l'une quelconque des revendications 6 à 16, **caractérisé en ce que** le catalyseur de silicalite de titane est présent en tant que lit fixe.

20. Procédé d'époxydation catalytique du propène dans lequel
a) dans une étape de réaction, le propène est mis en réaction avec du peroxyde d'hydrogène aqueux dans le méthanol en présence d'un catalyseur de silicalite de titane,
b) le flux de produit provenant de l'étape de réaction passe en option à une étape de décompression, et
c) le flux de produit est alors séparé, sans séparation par distillation antérieure, dans un pré-évaporateur ayant moins de 10 étapes de séparation théoriques à une pression de 1,5 à 2,9 bars, en un produit de tête contenant du propène, de l'oxyde de propène et du méthanol et en un produit de fond contenant du méthanol et de l'eau, 20 à 60% de la quantité totale de méthanol introduite avec le flux de produit étant éliminée avec le produit de tête et le résidu restant dans le produit de fond, le pré-évaporateur étant chauffé à l'aide de la chaleur de condensation des vapeurs résultant des étapes de distillation de phases subséquentes de traitement,
d) le produit de tête de l'étape c) est au moins partiellement condensé, le condensat contenant, en option après élimination par stripage du propène et tout propane présent,
0 - 12 % en poids de propène
0 - 5 % en poids de propane
15 - 75 % en poids d'oxyde de propène
25 - 85 % en poids de méthanol et
0 - 8 % en poids d'eau, et
e) le condensat, de l'étape d) est soumis à une distillation extractive, dans laquelle
e1) le condensat est ajouté à une section médiane d'une colonne de distillation extractive,
e2) un solvant polaire à fonctionnalité hydroxyle et ayant un point d'ébullition qui est supérieur à celui du méthanol est ajouté à la colonne de distillation extractive en un point au-dessus du point au niveau duquel le condensat pénètre,
e3) de l'oxyde de propène est éliminé par distillation à la tête de la colonne,
e4) un produit de fond contenant du méthanol et le solvant polaire est éliminé, et
f) une partie du produit de fond de l'étape c), en option après élimination partielle de l'eau, est recyclée à l'étape de réaction a).

21. Procédé selon la revendication 20, **caractérisé en ce que** le solvant polaire est sélectionné parmi l'eau, les glycols, les éthers du glycol et des mélanges de ces derniers.
